# EUROPEAN PATENT APPLICATION

(11) **EP 3 023 505 A1**
(43) Date of publication of application: **25.05.2016**
(21) Application number: 15195340.3
(22) Date of filing: 19.11.2015
(51) Int. Cl.: C12Q 1/68

(54) **NUCLEIC ACID AMPLIFICATION METHOD**

(30) Priority: 20.11.2014 JP 2014235330
(71) Applicant: Seiko Epson Corporation, Tokyo 163 (JP)
(72) Inventor: Uehara, Masayuki, Nagano, 390-8621 (JP)
(74) Representative: Hoffmann Eitle

(57) **Abstract**

A nucleic acid amplification method includes: heating a first region of a nucleic acid amplification reaction vessel filled with a nucleic acid amplification reaction mixture and a liquid which has a specific gravity different from that of the nucleic acid amplification reaction mixture and is immiscible with the nucleic acid amplification reaction mixture to a first temperature; heating a second region of the nucleic acid amplification reaction vessel to a second temperature lower than the first temperature; switching over from a first arrangement in which the first region is located lower than the second region in the direction of the gravitational force to a second arrangement in which the second region is located lower than the first region in the direction of the gravitational force; and switching over from the second arrangement to the first arrangement, wherein the nucleic acid amplification reaction mixture contains a probe, and the probe contains an artificial nucleic acid.

## Description

### BACKGROUND

### 1. Technical Field

The present invention relates to a nucleic acid amplification method.

### 2. Related Art

In recent years, as a result of development of technologies utilizing genes, medical treatments utilizing genes such as gene diagnosis or gene therapy have been drawing attention. In addition, many methods using genes in determination of breed varieties or breed improvement have also been developed in agricultural and livestock industries. As technologies for utilizing genes, nucleic acid amplification technologies such as a PCR (Polymerase Chain Reaction) method have been widely used. In recent years, a PCR apparatus capable of obtaining a nucleic acid amplification reaction product in a short time has been developed (for example, JP-A-2012-115208).

The inventors of this invention found that in a nucleic acid amplification reaction using the PCR apparatus disclosed in JP-A-2012-115208, when the period of thermal denaturation and annealing/elongation is decreased, the efficiency of nucleic acid amplification is sometimes decreased. As a result of intensive studies for finding the cause and solving the problem, the inventors found that by using an artificial nucleic acid in the PCR apparatus, the nucleic acid amplification can be efficiently performed.

### SUMMARY

An advantage of some aspects of the invention is to provide a novel nucleic acid amplification method with which efficient nucleic acid amplification can be performed.

An aspect of the invention is directed to a nucleic acid amplification method including: heating a first region of a nucleic acid amplification reaction vessel filled with a nucleic acid amplification reaction mixture and a liquid which has a specific gravity smaller than that of the nucleic acid amplification reaction mixture and is immiscible with the nucleic acid amplification reaction mixture to a first temperature; heating a second region of the nucleic acid amplification reaction vessel to a second temperature lower than the first temperature; switching over from a first arrangement in which the first region is located lower than the second region in the direction of the gravitational force to a second arrangement in which the second region is located lower than the first region in the direction of the gravitational force; and switching over from the second arrangement to the first arrangement, wherein the nucleic acid amplification reaction mixture contains a probe, and the probe contains an artificial nucleic acid. The probe may have a larger binding force to a template nucleic acid than a probe containing only a natural nucleic acid. The artificial nucleic acid may be LNA, 3'-amino-2',4'-BNA, 2',4'-BNA^{COC}, 2',4' -BNA^{NC}, PNA, GNA, or TNA. The artificial nucleic acid may be LNA. The probe may be a labeled probe. The labeled probe may be an RI-labeled probe, a fluorescence-labeled probe, or an enzyme-labeled probe.

Another aspect of the invention is directed to a nucleic acid amplification reaction apparatus including: a fitting section capable of fitting a nucleic acid amplification reaction vessel filled with a nucleic acid amplification reaction mixture and a liquid which has a specific gravity smaller than that of the nucleic acid amplification reaction mixture and is immiscible with the nucleic acid amplification reaction mixture; a first heating section which heats a first region of the nucleic acid amplification reaction vessel to a first temperature; a second heating section which heats a second region of the nucleic acid amplification reaction vessel to a second temperature; and a driving mechanism which switches over between a first arrangement in which the first region is located lower than the second region in the direction of the gravitational force and a second arrangement in which the second region is located lower than the first region in the direction of the gravitational force, wherein the nucleic acid amplification reaction mixture contains a probe, and the probe contains an artificial nucleic acid.

According to the aspects of the invention, a novel nucleic acid amplification method with which efficient nucleic acid amplification can be performed can be provided.

### BRIEF DESCRIPTION OF THE DRAWINGS

The invention will be described with reference to the accompanying drawings, wherein like numbers reference like elements.
FIG. 1 is a cross-sectional view of a nucleic acid amplification reaction vessel to be used in one embodiment of the invention. The arrow g indicates the direction of the gravitational force.
FIGS. 2A and 2B are perspective views of an up-and-down type PCR apparatus to be used in one embodiment of the invention. FIG. 1A shows a state in which a lid is closed and FIG. 1B shows a state in which the lid is opened.
FIG. 3 is an exploded perspective view of a main body of the up-and-down type PCR apparatus to be used in one embodiment of the invention.
FIGS. 4A and 4B are cross-sectional views schematically showing the cross section taken along the line A-A of FIG. 2A of the main body of the up-and-down type PCR apparatus to be used in one embodiment of the invention. FIG. 4A shows a first arrangement and FIG. 4B shows a second arrangement.
FIG. 5 is a graph showing the relationship between the measurement of fluorescence brightness which indicates the amount of a nucleic acid amplification reaction product and the thermal cycle number in one embodiment of the invention and Comparative Example.

### DESCRIPTION OF EXEMPLARY EMBODIMENTS

The object, features, and advantages of the invention as well as the idea thereof will be apparent to those skilled in the art from the description given herein, and the invention can be easily reproduced by those skilled in the art based on the description given herein. It is to be understood that the embodiments, specific examples, etc. of the invention described below are to be taken as preferred embodiments of the invention, and are presented for illustrative or explanatory purposes and are not intended to limit the invention. It is further apparent to those skilled in the art that various changes and modifications may be made based on the description given herein within the intent and scope of the invention disclosed herein.

### (1) Nucleic Acid Amplification Method

A nucleic acid amplification method according to the invention includes: heating a first region of a nucleic acid amplification reaction vessel filled with a nucleic acid amplification reaction mixture and a liquid which has a specific gravity smaller than that of the nucleic acid amplification reaction mixture and is immiscible with the nucleic acid amplification reaction mixture to a first temperature; heating a second region of the nucleic acid amplification reaction vessel to a second temperature lower than the first temperature; switching over from a first arrangement in which the first region is located lower than the second region in the direction of the gravitational force to a second arrangement in which the second region is located lower than the first region in the direction of the gravitational force; and switching over from the second arrangement to the first arrangement, wherein the nucleic acid amplification reaction mixture contains a probe, and the probe contains an artificial nucleic acid.

The nucleic acid amplification reaction mixture may contain a reagent for a nucleic acid amplification reaction and a target nucleic acid to be amplified. Examples of the target nucleic acid include a DNA prepared from a specimen such as blood, urine, saliva, spinal fluid, or a tissue and a cDNA obtained by reverse transcription of an RNA prepared from any of the above specimens. The reagent for a nucleic acid amplification reaction may contain primers for amplifying a target nucleic acid, a buffer, a polymerase, dNTPs, MgCl₂, a fluorescent label for detecting an amplification product of the target nucleic acid, and the like. The DNA polymerase is not particularly limited, but is preferably a heat-resistant enzyme or an enzyme for use in PCR, and there are a great number of commercially available products, for example, Taq polymerase, Tfi polymerase, Tth polymerase, modified forms thereof, and the like, however, a DNA polymerase capable of performing hot start PCR is preferred. The concentration of dNTPs or a salt may be set to a concentration suitable for the enzyme to be used, however, the concentration of dNTPs may be set to generally 10 to 1000 µM, and preferably 100 to 500 µM, the concentration of Mg²⁺ may be set to generally 1 to 100 mM, and preferably 5 to 10 mM, and the concentration of Cl⁻ may be set to generally 1 to 2000 mM, and preferably 200 to 700 mM. The total ion concentration is not particularly limited, but may be higher than 50 mM, and is preferably higher than 100 mM, more preferably higher than 120 mM, further more preferably higher than 150 mM, and still further more preferably higher than 200 mM. The upper limit thereof is preferably 500 mM or less, more preferably 300 mM or less, and further more preferably 200 mM or less. Each oligonucleotide for the primer is used at 0.1 to 10 µM, and preferably at 0.1 to 1 µM.

In the invention, the nucleic acid amplification reaction mixture contains a probe containing an artificial nucleic acid. The "artificial nucleic acid" as used herein refers to a nucleic acid molecule which can bind to a base of a DNA or an RNA through a hydrogen bond and is other than natural nucleic acid molecules. The type of the artificial nucleic acid to be used in the invention is not particularly limited, but is particularly preferably an artificial nucleic acid which increases the Tm value of a hybrid between a probe and a strand complementary to the probe and enhances the binding force of the probe to a template nucleic acid as compared with a probe composed only of a natural nucleic acid, and is preferably 2',4'-BNA (2'-O,4'-C-methano-bridged nucleic acid, also called "LNA (Locked Nucleic Acid)") in which an oxygen atom at position 2' and a carbon atom at position 4' of a ribose ring of a nucleic acid are methylene-bridged. The chemical formula of LNA is shown below.

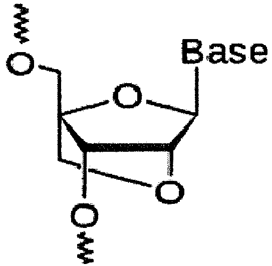

In the above formula, examples of the base include T (thymine), C(cytosine), G(guanine), A (adenine), U(uracil), and I (inosine), but the base is not particularly limited. The base may be a base modified by methylation, acetylation, or the like. Further, an LNA analogue obtained by modifying LNA may be used, and examples thereof include 3'-amino-2',4'-BNA, 2',4'-BNA^{COC}, and 2',4'-BNA^{NC}. Alternatively, PNA (Peptide Nucleic Acid), GNA (Glycol Nucleic Acid), or TNA (Threose Nucleic Acid), or an analogue obtained by modifying any of these may be used. Also in the LNA analogue, and PNA, GNA, TNA, and analogues thereof, the base is not limited in the same manner as in LNA.

The number of artificial nucleic acids contained in the probe is not particularly limited, and may be arbitrarily set according to the sequence of a target nucleic acid or a probe, or the like. One probe may contain multiple types of artificial nucleic acids.

The probe containing an artificial nucleic acid is preferably labeled for detecting the amplification of a target nucleic acid. The type of the label is not particularly limited and may be arbitrarily selected from commercially available products, for example, a fluorescent label such as TaqMan (registered trademark) MGB probe (Applied Biosystems), an intercalater such as SYBR Green (registered trademark), a radioactive material label such as a radioisotope (RI) typified by ³²P, an enzyme label typified by biotin or digoxigenin, etc. according to the purpose. Also the production method for the probe containing an artificial nucleic acid is not particularly limited, and the probe containing an artificial nucleic acid can be produced by a known method.

Here, the "Tm value" refers to a temperature at which 50% of the double-stranded nucleic acids are denatured into single-stranded nucleic acids, that is, a melting temperature. The calculation method for the Tm value is not particularly limited, and generally, the Tm value can be calculated by a GC% method, a nearest neighbor method, or a Wallace method alone, or by combining these methods.

By using such a probe containing an artificial nucleic acid, as compared with the case where a probe containing only a natural nucleic acid is used, an amplification curve starts to rise faster, and a larger amount of a nucleic acid amplification reaction product is obtained at the same cycle number, and thus, also a larger amount of a final product is obtained. In other words, when the denaturation temperature, the annealing temperature, and the elongation temperature are fixed, by using a probe containing an artificial nucleic acid, as compared with the case where a probe containing only a natural nucleic acid is used, the same amount of an amplification product is obtained in a shorter time.

The nucleic acid amplification reaction mixture may further contain a surfactant. The surfactant is not particularly limited, however, examples thereof include NP-40, Triton X-100, and Tween 20. The concentration of the surfactant is not particularly limited, but is preferably a concentration which does not inhibit the nucleic acid amplification reaction, and may be from 0.001% to 0.1% or less, and is preferably from 0.002% to 0.02%, and most preferably from 0.005% to 0.01%. The surfactant may be a carry-over from a stock solution of the enzyme described above, however, a surfactant solution may be added to the nucleic acid amplification reaction mixture independently of the stock solution of the enzyme.

By using a liquid which is immiscible with the reaction mixture 140 as the liquid 130, when the reaction mixture 140 is placed in the vessel 150, the reaction mixture 140 and the liquid 130 are phase-separated from each other, and therefore, the reaction mixture 140 can be formed into a liquid droplet in the liquid 130. In this manner, the reaction mixture 140 is maintained in the form of a liquid droplet in the liquid 130.

The liquid 130 is preferably a liquid having a specific gravity smaller than that of the reaction mixture 140. In this case, when the reaction mixture 140 is placed in the liquid 130, the liquid droplet of the reaction mixture 140 has a specific gravity larger than that of the liquid 130, and therefore moves in the direction of the gravitational force by the action of gravity. Further, the liquid 130 may be a liquid having a specific gravity larger than that of the reaction mixture 140. In this case, the liquid droplet of the reaction mixture 140 has a specific gravity smaller than that of the liquid 130, and therefore moves in the direction opposite to the direction of the gravitational force by the action of gravity.

The liquid 130 preferably contains an oil, and for example, a silicone oil or a mineral oil can be used. Here, the "silicone" means an oiligomer or a polymer having a siloxane bond as a main skeleton. In this specification, among silicones, a silicone in the form of a liquid in a temperature range in which the silicone is used in a thermal cycling treatment is particularly referred to as "silicone oil". Further, in this specification, an oil which is purified from petroleum and is in the form of a liquid in a temperature range in which the oil is used in a thermal cycling treatment is referred to as "mineral oil". These oils have high stability against heat, and for example, products having a viscosity of 5 x 10³ Nsm⁻² or less are also easily available, and therefore, these oils are preferred for use in up-and-down type PCR.

Examples of the silicone oil include dimethyl silicone oils such as KF-96L-0.65cs, KF-96L-1cs, KF-96L-2cs, KF-96L-5cs (manufactured by Shin-Etsu Silicone Co., Ltd.), SH200 C FLUID 5 CS (manufactured by Dow Corning Toray Co. , Ltd.), TSF451-5A, and TSF451-10 (manufactured by Momentive Performance Materials Japan LLC). Examples of the mineral oil include oils containing alkane having about 14 to 20 carbon atoms as a principal component, and specific examples thereof include n-tetradecane, n-pentadecane, n-hexadecane, n-heptadecane, n-octadecane, n-nonadecane, and n-tetracosane.

The liquid 130 may contain an additive. As the additive, for example, a modified silicone oil such as X-22-160AS, X-22-3701E, KF-857, KF-859, KF-862, KF-867, KF-6017, or KF-8005 (Shin-Etsu Silicone Co., Ltd.), a silicone resin such as SR1000, SS4230, SS4267, or YR3370 (Momentive Performance Materials, Inc.), a fluoro-modified silicone resin such as XS66-C1191 (Momentive Performance Materials, Inc.), or the like, and other than these, a modified silicone oil such as TSF4703, TSF4708, XF42-C5196, or XF42-C5197 (Momentive Performance Materials, Inc.) can be used. The concentration of the additive is not particularly limited, but can be determined in consideration of the structure, material, shape, or the like of the vessel. For example, the concentration thereof is preferably 1% (v/v) or more and 50% (v/v) or less, more preferably 2% (v/v) or more and 20% (v/v) or less, and further more preferably 5% (v/v).

Hereinafter, as one embodiment of the nucleic acid amplification method according to the invention, a nucleic acid amplification method using shuttle PCR (two-stage temperature PCR) will be described. The shuttle PCR is a method of amplifying a nucleic acid in a reaction mixture by subjecting the reaction mixture to a two-stage temperature treatment at a high temperature and a low temperature repeatedly. In the treatment at a high temperature, denaturation of a double-stranded DNA occurs and in the treatment at a low temperature, annealing (a reaction in which a primer binds to a single-stranded DNA) and elongation (a reaction in which a complementary strand to the DNA is synthesized by using the primer as a starting point) occur.

In the method according to the invention, a nucleic acid amplification reaction vessel filled with a nucleic acid amplification reaction mixture and a liquid which has a specific gravity smaller than that of the nucleic acid amplification reaction mixture and is immiscible with the nucleic acid amplification reaction mixture is used, and a first region of the nucleic acid amplification reaction vessel is heated to a first temperature, and a second region of the nucleic acid amplification reaction vessel is heated to a second temperature lower than the first temperature. Here, a temperature gradient in which the temperature decreases from the first region to the second region is formed. The first temperature is set to a temperature suitable for the denaturation of a double-stranded DNA and the second temperature is set to a temperature suitable for annealing and elongation. Then, the denaturation of a double-stranded DNA is performed by maintaining the first arrangement in which the first region is located lower than the second region in the direction of the gravitational force for a predetermined period, and annealing and elongation are performed by maintaining the second arrangement in which the second region is located lower than the first region in the direction of the gravitational force for a predetermined period. By switching over between the first arrangement and the second arrangement at fixed times, a nucleic acid is amplified. Due to a difference in specific gravity between the nucleic acid amplification reaction mixture and the liquid, the nucleic acid amplification reaction mixture is present in the first region in the first arrangement and is present in the second region in the second arrangement.

In general, in shuttle PCR, the high temperature is a temperature between 80°C and 100°C and the low temperature is a temperature between 50°C and 70°C. The treatments at the respective temperatures are performed for a predetermined period, and a period in which the reaction mixture is maintained at a high temperature is generally shorter than a period in which the reaction mixture is maintained at a low temperature. For example, the period of the treatment at a high temperature may be set to about 1 to 10 seconds, and the period of the treatment at a low temperature may be set to about 10 to 60 seconds, or a period longer than this range may be adopted depending on the condition of the reaction. However, the period of the treatment in which the reaction mixture is maintained at a low temperature is preferably shorter, and may be 18 seconds or less, and is preferably 12 seconds or less, and more preferably 6 seconds or less.

The appropriate period, temperature, cycle number (the number of repetitions of the treatment at a high temperature and the treatment at a low temperature) vary depending on the type or amount of a reagent to be used, and therefore, it is preferred to determine an appropriate protocol in consideration of the type of a reagent or the amount of the nucleic acid amplification reaction mixture before performing the reaction.

In this embodiment, the description has been made by using, as the nucleic acid amplification method, shuttle PCR in which the nucleic acid amplification reaction is performed at two temperatures, however, a PCR method (three-stage temperature PCR) in which the temperature is changed in thermal denaturation, annealing, and elongation reactions may be adopted. In this case, in addition to an annealing temperature, an elongation reaction temperature is set, and after the nucleic acid amplification reaction mixture is maintained at the annealing temperature, it may be maintained at the elongation reaction temperature for a predetermined period of time.

### (2) Nucleic Acid Amplification Reaction Vessel

As one embodiment of the invention, a nucleic acid amplification reaction vessel to be used in the method according to the invention will be described below. The nucleic acid amplification reaction vessel to be used in the method according to the invention is a nucleic acid amplification reaction vessel which has a hermetically sealed vessel containing a nucleic acid amplification reaction mixture and a liquid having a specific gravity different from that of the nucleic acid amplification reaction mixture and is immiscible with the nucleic acid amplification reaction mixture, wherein the nucleic acid amplification reaction mixture is in the form of a liquid droplet and the liquid contains an oil and an additive.

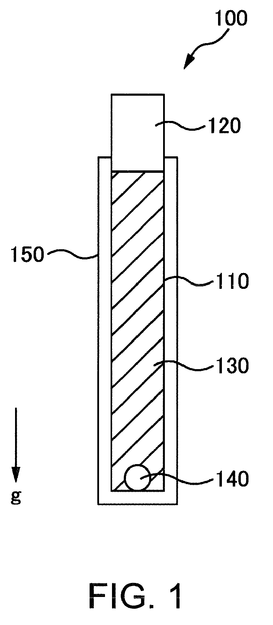

FIG. 1 is a cross-sectional view of a nucleic acid amplification reaction vessel 100. FIG. 1 shows a state in which a reaction mixture is placed in the nucleic acid amplification reaction vessel.

The nucleic acid amplification reaction vessel 100 to be used in the invention is configured to include a vessel 150 and a sealing section 120. The size and shape of the nucleic acid amplification reaction vessel 100 are not particularly limited, but may be designed in consideration of, for example, at least one of the amount of a liquid 130 which is immiscible with a nucleic acid amplification reaction mixture 140 (hereinafter also referred to as "reaction mixture 140"), the thermal conductivity thereof, the shapes of the vessel 150 and the sealing section 120, and the ease of handling thereof. The liquid 130 and the nucleic acid amplification reaction mixture 140 are selected from those described in "(1) Nucleic Acid Amplification Method".

The vessel 150 of the nucleic acid amplification reaction vessel 100 can be formed from a transparent material. According to this, the movement of the reaction mixture 140 in the vessel 150 can be observed from the outside of the nucleic acid amplification reaction vessel 100, or the vessel 150 can be used in an application in which the measurement or the like is performed from the outside of the vessel 150 such as real-time PCR. The term "transparent" as used herein refers to a condition in which the visibility can be ensured to such an extent that the reaction mixture 140 in the vessel 150 can be observed from the outside of the vessel 150, and it is not necessary that the entire nucleic acid amplification reaction vessel 100 should be transparent as long as this condition is met.

The application of the nucleic acid amplification reaction vessel 100 is not particularly limited, however, for example, in the case where the nucleic acid amplification reaction vessel 100 is used in an application with a fluorescence measurement such as real-time PCR, the vessel 150 is desirably formed from a material with a low autofluorescence. The vessel 150 is preferably formed from a material which can withstand heating in PCR. Further, the material of the vessel 150 is preferably a material, on which nucleic acids or proteins are less adsorbed, and which does not inhibit the enzymatic reaction by a polymerase or the like. The material which satisfies these conditions is not particularly limited, and for example, polypropylene, polyethylene, a cycloolefin polymer (for example, ZEONEX (registered trademark) 480R), a heat-resistant glass (for example, PYREX (registered trademark) glass), or the like, or a composite material thereof may be used, however, polypropylene is preferred.

In the nucleic acid amplification reaction vessel 100 shown in FIG. 1, the vessel 150 is formed into a cylindrical shape, and the direction of the center axis (the vertical direction in Fig. 1) coincides with the longitudinal direction. The vessel 150 used here is preferably a tube and may be a tube for a microcentrifuge or a tube designed for PCR. Since the vessel 150 has a shape with a longitudinal direction, in other words, an elongated shape, for example, in the case where the temperature of the nucleic acid amplification reaction vessel 100 is controlled so that regions having different temperatures are formed in the liquid 130 in the vessel 150 using an up-and-down type thermal cycler, which will be described later, the distance between the regions having different temperatures is easily increased, According to this, it becomes easy to control the temperature of the liquid 130 to be different from region to region in the vessel 150, and therefore, a thermal cycle suitable for PCR can be realized. The "up-and-down type thermal cycler" is an apparatus which realizes a thermal cycle by forming at least two temperature regions in a liquid filled in the vessel 150 and allowing the reaction mixture 140 which is phase-separated from the liquid to move reciprocatingly between these temperature regions.

The shape of the vessel 150 is not particularly limited as long as it has a longitudinal direction, however, in the case where the vessel 150 is used for an up-and-down type PCR apparatus, it is preferred that the shape is a substantially cylindrical shape and the ratio of the inner diameter D to the length L in the longitudinal direction is in the range of 1:5 to 5:20. It is more preferred that the inner diameter D is from 1.5 to 2 mm, and the length L is from 10 to 20 mm.

The vessel 150 has an opening section and the sealing section 120 which seals the opening section, and in the vessel 150, the reaction mixture 140 and the liquid 130 which has a specific gravity different from that of the reaction mixture 140 and is phase-separated from the reaction mixture 140 are contained. It is preferred that in the case where the opening section is sealed by the sealing section 120, air does not remain in the vessel 150. It is because if an air bubble remains in the vessel 150, the movement of the reaction mixture 140 may be hindered. The sealing section 120 can be formed from the same material as that of the vessel 150. The structure of the sealing section 120 may be any as long as it can hermetically seal the vessel 150, and can be a structure of, for example, a screw cap, a plug, an inlay, or the like. In FIG. 1, the sealing section 120 has a structure of a screw cap.

### (3) Configuration of Nucleic Acid Amplification Reaction Apparatus

In this embodiment, as the nucleic acid amplification reaction vessel to be used for performing a nucleic acid amplification reaction, a nucleic acid amplification reaction tube 100 in the form of a tube is used. Hereinafter, by taking PCR as one example of the nucleic acid amplification reaction, one example of a nucleic acid amplification reaction apparatus (hereinafter also referred to as "up-and-down type PCR apparatus") suitable for the nucleic acid amplification reaction tube 100 will be described in detail. The up-and-down type PCR apparatus is disclosed in detail in JP-A-2012-115208.

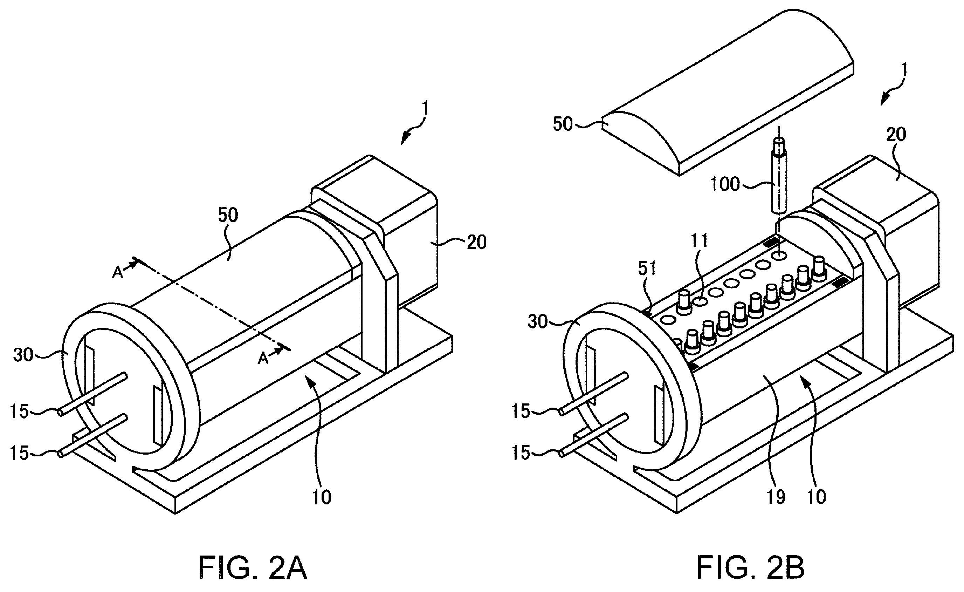

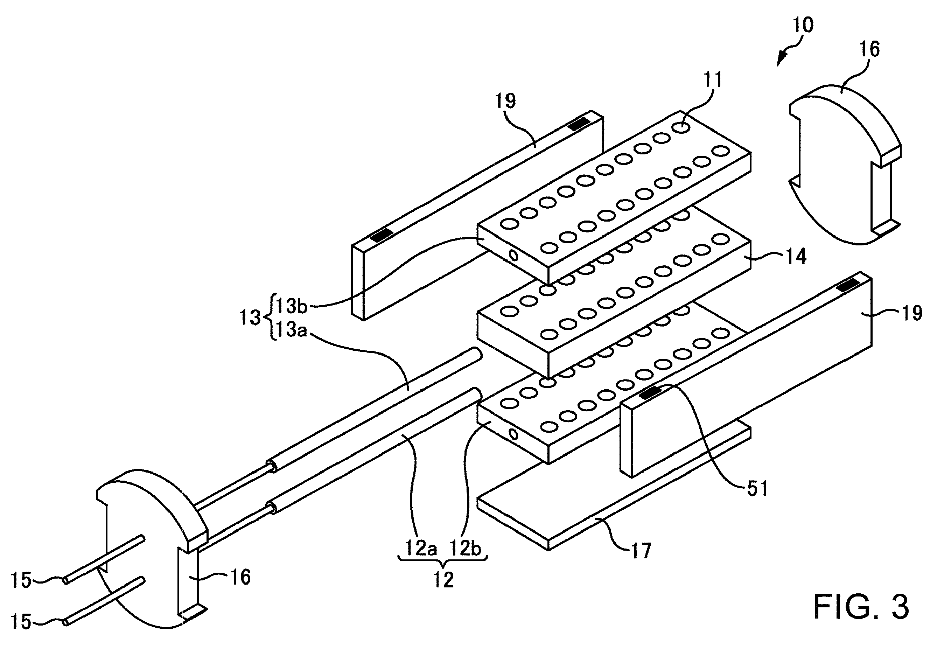

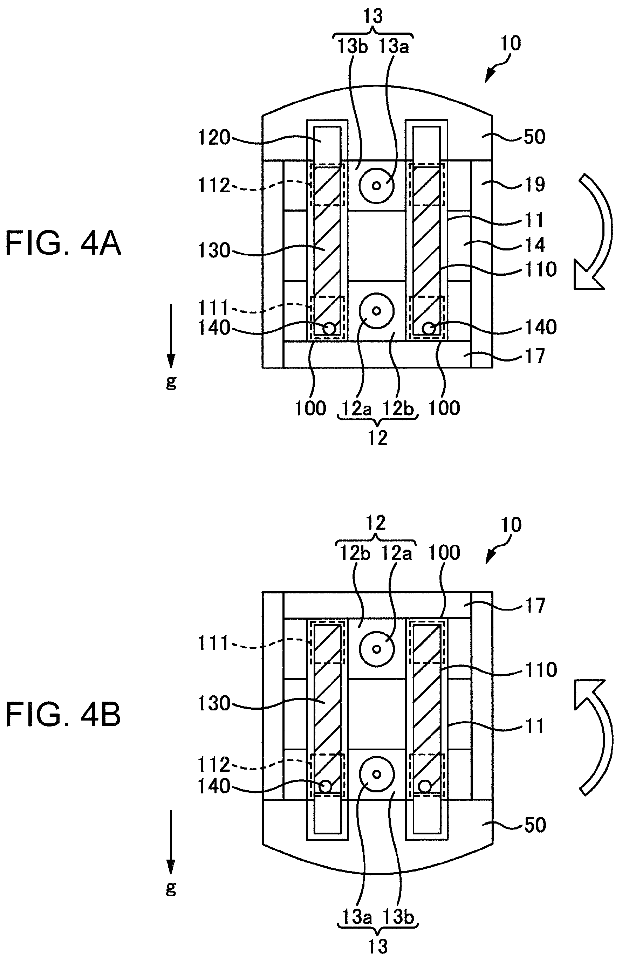

FIGS. 2A and 2B show one example of an up-and-down type PCR apparatus 1. FIG. 2A shows a state in which a lid 50 of the up-and-down type PCR apparatus 1 is closed, and FIG. 2B shows a state in which the lid 50 of the up-and-down type PCR apparatus 1 is opened and the nucleic acid amplification reaction tube 100 is fitted in a fitting section 11. FIG. 3 is an exploded perspective view of a main body 10 of the up-and-down type PCR apparatus 1 according to the embodiment. FIGS. 4A and 4B are cross-sectional views schematically showing the cross section taken along the line A-A of FIG. 2A of the main body 10 of the up-and-down type PCR apparatus 1 according to the embodiment.

This up-and-down type PCR apparatus 1 includes the main body 10 and a driving mechanism 20 as shown in FIG. 2A. As shown in FIG. 3, the main body 10 includes the fitting section 11, a first heating section 12, and a second heating section 13. A spacer 14 is provided between the first heating section 12 and the second heating section 13. In the main body 10 of this embodiment, the first heating section 12 is disposed on the bottom plate 17 side, and the second heating section 13 is disposed on the lid 50 side. In the main body 10 of this embodiment, the first heating section 12, the second heating section 13, and the spacer 14 are fixed by a flange 16, the bottom plate 17, and a fixing plate 19.

The fitting section 11 is configured such that the nucleic acid amplification reaction tube 100, which will be described later, is fitted therein. As shown in FIG. 2B and FIG. 3, the fitting section 11 of this embodiment has a slot structure in which the nucleic acid amplification reaction tube 100 is inserted and fitted, and is configured such that the nucleic acid amplification reaction tube 100 is inserted into a hole penetrating a first heat block 12b of the first heating section 12, the spacer 14, and a second heat block 13b of the second heating section 13. The number of the fitting sections 11 may be more than one, and in the example shown in FIG. 2B, twenty fitting sections 11 are provided for the main body 10.

This up-and-down type PCR apparatus 1 includes a structure in which the nucleic acid amplification reaction tube 100 is held at a predetermined position with respect to the first heating section 12 and the second heating section 13. More specifically, as shown in FIGS. 4A and 4B, in a flow channel 110 constituting the nucleic acid amplification reaction tube 100, which will be described later, a first region 111 can be heated by the first heating section 12 and a second region 112 can be heated by the second heating section 13. In this embodiment, a structure that defines the position of the nucleic acid amplification reaction tube 100 is the bottom plate 17, and as shown in FIG. 4A, by inserting the nucleic acid amplification reaction tube 100 to a position where the tube is in contact with the bottom plate 17, the nucleic acid amplification reaction tube 100 can be held at a predetermined position with respect to the first heating section 12 and the second heating section 13.

When the nucleic acid amplification reaction tube 100 is fitted in the fitting section 11, the first heating section 12 heats the first region 111 of the nucleic acid amplification reaction tube 100, which will be described later, to a first temperature. In the example shown in FIG. 4A, in the main body 10, the first heating section 12 is disposed at a position where the first region 111 of the nucleic acid amplification reaction tube 100 is heated.

The first heating section 12 may include a mechanism that generates heat and a member that transfers the generated heat to the nucleic acid amplification reaction tube 100. In the example shown in FIG. 3, the first heating section 12 includes a first heater 12a and a first heat block 12b. In this embodiment, the first heater 12a is a cartridge heater and is connected to an external power source (not shown) through a conductive wire 15. The first heater 12a is inserted into the first heat block 12b, and the first heat block 12b is heated by heat generated by the first heater 12a. The first heat block 12b is a member that transfers heat generated by the first heater 12a to the nucleic acid amplification reaction tube 100. In this embodiment, the first heat block 12b is a block made of aluminum.

When the nucleic acid amplification reaction tube 100 is fitted in the fitting section 11, the second heating section 13 heats the second region 112 of the nucleic acid amplification reaction tube 100 to a second temperature different from the first temperature. In the example shown in FIG. 4A, in the main body 10, the second heating section 13 is disposed at a position where the second region 112 of the nucleic acid amplification reaction tube 100 is heated. As shown in FIG. 3, the second heating section 13 includes a second heater 13a and the second heat block 13b. The second heating section 13 is configured in the same manner as the first heating section 12 except that the region of the nucleic acid amplification reaction tube 100 to be heated and the heating temperature are different from those for the first heating section 12.

In this embodiment, the temperatures of the first heating section 12 and the second heating section 13 are controlled by a temperature sensor (not shown) and a control section (not shown), which will be described later. The temperatures of the first heating section 12 and the second heating section 13 are preferably set so that the nucleic acid amplification reaction tube 100 is heated to a desired temperature. In this embodiment, by controlling the first heating section 12 at the first temperature and the second heating section 13 at the second temperature, the first region 111 of the nucleic acid amplification reaction tube 100 can be heated to the first temperature, and the second region 112 can be heated to the second temperature. The temperature sensor in this embodiment is a thermocouple.

The driving mechanism 20 is a mechanism that controls the fitting section 11, the first heating section 12, and the second heating section 13, and by the driving mechanism, the arrangement of the first region and the second region is controlled. In this embodiment, the driving mechanism 20 includes a motor (not shown) and a drive shaft (not shown), and the drive shaft is connected to the flange 16 of the main body 10. The drive shaft in this embodiment is provided perpendicular to the longitudinal direction of the fitting section 11, and when the motor is activated, the main body 10 is rotated about the drive shaft as the axis of rotation.

The up-and-down type PCR apparatus 1 of this embodiment includes the control section (not shown). The control section controls at least one of the first temperature, the second temperature, a first period, a second period, and the cycle number of thermal cycles, which will be described later. In the case where the control section controls the first period or the second period, the control section controls the operation of the driving mechanism 20, thereby controlling the period in which the fitting section 11, the first heating section 12, and the second heating section 13 are held in a predetermined arrangement. The control section may have mechanisms different from item to item to be controlled, or may control all items collectively. However, the control section in the up-and-down type PCR apparatus 1 of this embodiment is an electronic control system and controls all of the above-mentioned items. The control section of this embodiment includes a processor such as a CPU (not shown) and a storage device such as an ROM (Read Only Memory) or an RAM (Random Access Memory). In the storage device, various programs, data, etc. for controlling the above-mentioned respective items are stored. Further, the storage device has a work area for temporarily storing data in processing, processing results, etc. of various processes.

As shown in the example of FIG. 3 and FIG. 4A, in the main body 10 of this embodiment, the spacer 14 is provided between the first heating section 12 and the second heating section 13. The spacer 14 of this embodiment is a member that holds the first heating section 12 or the second heating section 13. In this embodiment, the spacer 14 is a heat insulating material, and in the example shown in FIG. 4A, the fitting section 11 penetrates the spacer 14.

The main body 10 of this embodiment includes the fixing plate 19. The fixing plate 19 is a member that holds the fitting section 11, the first heating section 12, and the second heating section 13. In the example shown in FIG. 2B and FIG. 3, two fixing plates 19 are fitted in the flanges 16, and the first heating section 12, the second heating section 13, and the bottom plate 17 are fixed by the fixing plates 19.

The up-and-down type PCR apparatus 1 of this embodiment includes the lid 50. In the example shown in FIG. 2A and FIG. 4A, the fitting section 11 is covered with the lid 50. The lid 50 may be fixed to the main body 10 by a fixing section 51. In this embodiment, the fixing section 51 is a magnet. As shown in the example of FIG. 2B and FIG. 3, a magnet is provided on a surface of the main body 10 which comes into contact with the lid 50. Although not shown in FIG. 2B and FIG. 3, a magnet is provided also for the lid 50 at a place where the magnet of the main body 10 comes into contact. When the fitting section 11 is covered with the lid 50, the lid 50 is fixed to the main body 10 by a magnetic force.

It is preferred that the fixing plate 19, the bottom plate 17, the lid 50, and the flange 16 are formed using a heat insulating material.

### (4) Thermal Cycling Treatment Using Up-and-down type PCR Apparatus

FIGS. 4A and 4B are cross-sectional views schematically showing the cross section taken along the line A-A of FIG. 2A of the up-and-down type PCR apparatus 1. FIGS. 4A and 4B show a state in which the nucleic acid amplification reaction tube 100 is fitted in the up-and-down type PCR apparatus 1. FIG. 4A shows a first arrangement and FIG. 4B shows a second arrangement. Hereinafter, as one embodiment of the nucleic acid amplification method according to the invention, a thermal cycling treatment using the up-and-down type PCR apparatus 1 according to the embodiment in the case of using the nucleic acid amplification reaction tube 100 will be described.

As shown in the example of FIG. 1, the nucleic acid amplification reaction tube 100 according to the embodiment includes a flow channel 110 and a sealing section 120. The flow channel 110 is filled with a reaction mixture 140 and a liquid 130 which has a specific gravity smaller than that of the reaction mixture 140 and is immiscible with the reaction mixture 140, and sealed with the sealing section 120.

The flow channel 110 is formed such that the reaction mixture 140 moves in close proximity to opposed inner walls. Here, the phrase "opposed inner walls" of the flow channel 110 refers to two regions of a wall surface of the flow channel 110 having an opposed positional relationship. The phrase "in close proximity to" refers to a state in which the distance between the reaction mixture 140 and the wall surface of the flow channel 110 is close, and includes a case where the reaction mixture 140 is in contact with the wall surface of the flow channel 110. Therefore, the phrase "the reaction mixture 140 moves in close proximity to opposed inner walls" refers to that "the reaction mixture 140 moves in a state of being close in distance to both of the two regions of a wall surface of the flow channel 110 having an opposed positional relationship", that is, the reaction mixture 140 moves along the opposed inner walls.

In the example shown in Fig. 1, the outer shape of the nucleic acid amplification reaction tube 100 is a cylindrical shape, and the flow channel 110 is formed in the direction of the center axis (the vertical direction in Fig. 1) therein. The shape of the flow channel 110 is a cylindrical shape having a circular cross section perpendicular to the longitudinal direction of the flow channel 110, that is, perpendicular to the direction in which the reaction mixture 140 moves in a region in the flow channel 110 (this cross section is defined as the "cross section" of the flow channel 110). Therefore, in the nucleic acid amplification reaction tube 100 of this embodiment, the opposed inner walls of the flow channel 110 are regions including two points on the wall surface of the flow channel 110 constituting the diameter of the cross section of the flow channel 110, and the reaction mixture 140 moves in the longitudinal direction of the flow channel 110 along the opposed inner walls.

The first region 111 of the nucleic acid amplification reaction tube 100 is a partial region of the flow channel 110 which is heated to the first temperature by the first heating section 12. The second region 112 is a partial region of the flow channel 110, which is different from the first region 111, and is heated to the second temperature by the second heating section 13. In the nucleic acid amplification reaction tube 100 of this embodiment, the first region 111 is a region including one end portion in the longitudinal direction of the flow channel 110, and the second region 112 is a region including the other end portion in the longitudinal direction of the flow channel 110. In the example shown in FIGS. 4A and 4B, a region surrounded by the dotted line including an end portion on the proximal side of the sealing section 120 of the flow channel 110 is the second region 112, and a region surrounded by the dotted line including an end portion on the distal side of the sealing section 120 is the first region 111.

As shown in FIG. 1, the flow channel 110 contains the liquid 130 and a liquid droplet of the reaction mixture 140. The liquid 130 and the reaction mixture 140 are prepared according to the description of (1) Nucleic Acid Amplification Method.

Hereinafter, with reference to FIGS. 4A and 4B, the thermal cycling treatment using the up-and-down type PCR apparatus 1 according to the embodiment will be described. In FIGS. 4A and 4B, the direction indicated by the arrow g (in the downward direction in the drawing) is the direction of the gravitational force. In this embodiment, a case where shuttle PCR (two-stage temperature PCR) described in " (1) Nucleic Acid Amplification Method" is performed as an example of the thermal cycling treatment will be described. The respective steps described below show one example of the thermal cycling treatment, and according to need, the order of the steps may be changed, two or more steps may be performed continuously or concurrently, or a step may be added.

First, the nucleic acid amplification reaction tube 100 is fitted in the fitting section 11. In this embodiment, after the reaction mixture 140 is introduced into the flow channel 110 previously filled with the liquid 130, the nucleic acid amplification reaction tube 100 is sealed with the sealing section 120, and then fitted in the fitting section 11. The introduction of the reaction mixture 140 can be performed using a micropipette, an ink-jet dispenser, or the like. In a state in which the nucleic acid amplification reaction tube 100 is fitted in the fitting section 11, the first heating section 12 is in contact with the nucleic acid amplification reaction tube 100 at a position including the first region 111 and the second heating section 13 is in contact with the nucleic acid amplification reaction tube 100 at a position including the second region 112.

Here, the arrangement of the fitting section 11, the first heating section 12, and the second heating section 13 is the first arrangement. As shown in FIG. 4A, in the first arrangement, the first region 111 of the nucleic acid amplification reaction tube 100 is located in a lowermost portion of the flow channel 110 in the direction of the gravitational force. In the first arrangement, the first region 111 is located in a lowermost portion of the flow channel 110 in the direction of the gravitational force, and therefore, the reaction mixture 140 having a specific gravity larger than that of the liquid 130 is located in the first region 111. In this embodiment, after the nucleic acid amplification reaction tube 100 is fitted in the fitting section 11, the fitting section 11 is covered with the lid 50, and then the up-and-down type PCR apparatus 1 is operated.

Subsequently, the nucleic acid amplification reaction tube 100 is heated by the first heating section 12 and the second heating section 13. The first heating section 12 and the second heating section 13 heat different regions of the nucleic acid amplification reaction tube 100 to different temperatures. That is, the first heating section 12 heats the first region 111 to the first temperature, and the second heating section 13 heats the second region 112 to the second temperature. According to this, a temperature gradient in which the temperature gradually changes between the first temperature and the second temperature is formed between the first region 111 and the second region 112 of the flow channel 110. Here, a temperature gradient in which the temperature decreases from the first region 111 to the second region 112 is formed. The thermal cycling treatment of this embodiment is shuttle PCR, and therefore, the first temperature is set to a temperature suitable for the denaturation of a double-stranded DNA, and the second temperature is set to a temperature suitable for annealing and elongation.

Since the arrangement of the fitting section 11, the first heating section 12, and the second heating section 13 is the first arrangement, when the nucleic acid amplification reaction tube 100 is heated, the reaction mixture 140 is heated to the first temperature. When the first period has elapsed, the main body 10 is driven by the driving mechanism 20, and the arrangement of the fitting section 11, the first heating section 12, and the second heating section 13 is switched over from the first arrangement to the second arrangement. The second arrangement is an arrangement in which the second region 112 is located in a lowermost portion of the flow channel 110 in the direction of the gravitational force. In other words, the second region 112 is a region located in a lowermost portion of the flow channel 110 in the direction of the gravitational force when the fitting section 11, the first heating section 12, and the second heating section 13 are placed in a predetermined arrangement different from the first arrangement. In the up-and-down type PCR apparatus 1 of this embodiment, under the control of the control section, the driving mechanism 20 rotatively drives the main body 10. When the flanges 16 are rotatively driven by the motor by using the drive shaft as the axis of rotation, the fitting section 11, the first heating section 12, and the second heating section 13 which are fixed to the flanges 16 are rotated. Since the drive shaft is a shaft extending in the direction perpendicular to the longitudinal direction of the fitting section 11, when the drive shaft is rotated by the activation of the motor, the fitting section 11, the first heating section 12, and the second heating section 13 are rotated. In the example shown in FIGS. 4A and 4B, the main body 10 is rotated at 180°. By doing this, the arrangement of the fitting section 11, the first heating section 12, and the second heating section 13 is switched over from the first arrangement to the second arrangement.

Here, the positional relationship between the first region 111 and the second region 112 in the direction of the gravitational force is opposite to that of the first arrangement, and therefore, the reaction mixture 140 moves from the first region 111 to the second region 112 by the action of gravity. When the operation of the driving mechanism 20 is stopped after the arrangement of the fitting section 11, the first heating section 12, and the second heating section 13 has reached the second arrangement, the fitting section 11, the first heating section 12, and the second heating section 13 are held in the second arrangement. When the second period has elapsed in the second arrangement, the main body is rotated again. A nucleic acid amplification reaction is performed by rotation while switching over between the first arrangement and the second arrangement in this manner until completion of a predetermined number of cycles. An operation in which the first arrangement and the second arrangement are switched over once is defined as one cycle.

In this embodiment, as the nucleic acid amplification method, shuttle PCR is used, however, as described in "(1) Nucleic Acid Amplification Method", a PCR method (three-stage temperature PCR) in which the temperature is changed in thermal denaturation, annealing, and elongation reactions may be adopted. In this case, in addition to an annealing temperature, an elongation reaction temperature is set, and after the nucleic acid amplification reaction mixture is maintained at the annealing temperature, it may be maintained at the elongation reaction temperature for a predetermined period of time.

### Examples

Hereinafter, the invention will be described in more detail by showing Examples, however, the invention is not limited thereto.

By using the above-mentioned up-and-down type PCR apparatus, a nucleic acid amplification reaction was performed by using a probe containing an artificial nucleic acid as Example and a probe containing only a natural nucleic acid as Comparative Example, and also using 25 copies of a plasmid DNA of Mycoplasma pneumoniae as a template. In this Example, a fluorescence-labeled probe was used, and as the artificial nucleic acid, 2',4'-BNA (2'-O,4'-C-methano-bridged nucleic acid, also called "LNA (Locked Nucleic Acid)") was used.

The composition of the reaction mixture, the sequences of the primers, the sequences of the probes of Example and Comparative Example, and the conditions for the nucleic acid amplification reaction are as follows. As the liquid amount of each component described below, a liquid amount in 10 µL of the total amount of the reaction mixture is shown. (Composition of Reaction Mixture)

| | |
|---|---|
| Platinum Taq (polymerase) | 0.4 µL |
| dNTPs (10 mM) | 0.5 µL |
| 5x buffer (*) | 2.0 µL |
| Forward primer for detection of Mycoplasma (20 µM) | 0.8 µL |
| Reverse primer for detection of Mycoplasma (20 µM) | 0.8 µL |
| TaqMan probe for detection of Mycoplasma (10 µM) | 0.6 µL |
| Plasmid DNA of Mycoplasma pneumoniae | (Template) |
| | 1.0 µL |
| Water | 3.9 µL |
| | Total: 10 µL |

| | |
|---|---|
| * Composition of 5x buffer: MgCl₂: 25 mM, Tris-HCl (pH 9.0): 250 mM, KCl: 125 mM | |

### (Sequence of Primers)

Forward primer: 5' ATCCAGGTACGGGTGAAGACAC 3' (SEQ ID NO: 1)
Reverse primer: 5' CGCATCAACAAGTCCTAGCGAAC 3' (SEQ ID NO: 2)

### (Sequences of Probes)

Normal fluorescent probe (the probe containing only a natural nucleic acid, Comparative Example)
5' FAM-CGGGACGGAAAGACC-BHQ1 3' (SEQ ID NO: 3)
Artificial nucleic acid-introduced fluorescence-labeled probe (the probe containing an artificial nucleic acid, Example)
5' FAM-CGGGACGGAAAGACC-BHQ1 3' (SEQ ID NO: 4)
* The underlined part indicates that the nucleic acid is an artificial nucleic acid LNA.

### (Conditions for Nucleic Acid Amplification Reaction)

Hot start: 98°C, 10 sec
(1 cycle)
Thermal denaturation: 98°C, 4 sec
Annealing and elongation: 54°C, 6 sec
(50 cycles)

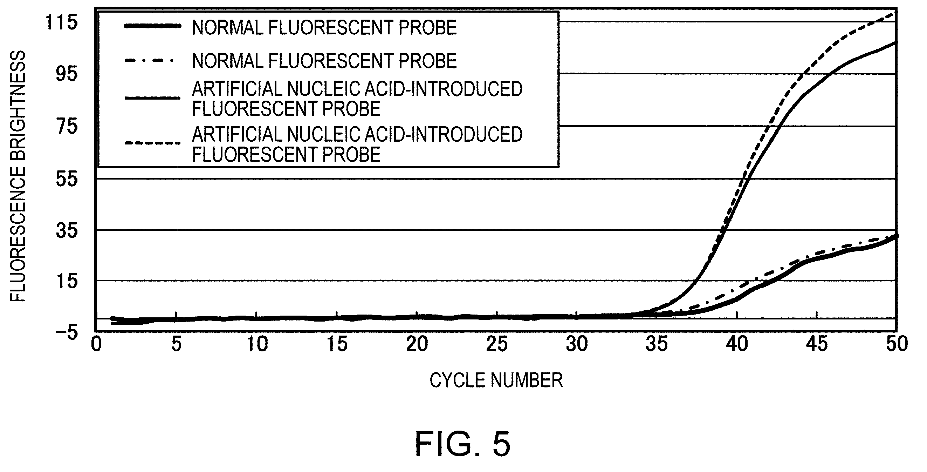

The results are shown in FIG. 5. The horizontal axis represents the cycle number and the vertical axis represents the measurement of fluorescence brightness which indicates the amount of a nucleic acid amplification reaction product.

As apparent from FIG. 5, as compared with the case where the probe containing only a natural nucleic acid was used, in the case where the probe containing an artificial nucleic acid was used, the amplification curve started to rise faster, and a larger amount of a nucleic acid amplification reaction product is obtained at the same cycle number, and thus, also a larger amount of a final product is obtained. Further, when the same amount of a product is synthesized, the synthesis can be achieved at a smaller cycle number in the case where the probe containing an artificial nucleic acid was used as compared with the case where the probe containing only a natural nucleic acid was used.

## Claims

1. A nucleic acid amplification method, comprising:
heating a first region of a nucleic acid amplification reaction vessel filled with a nucleic acid amplification reaction mixture and a liquid which has a specific gravity different from that of the nucleic acid amplification reaction mixture and is immiscible with the nucleic acid amplification reaction mixture to a first temperature;
heating a second region of the nucleic acid amplification reaction vessel to a second temperature lower than the first temperature;
switching over from a first arrangement in which the first region is located lower than the second region in the direction of the gravitational force to a second arrangement in which the second region is located lower than the first region in the direction of the gravitational force; and
switching over from the second arrangement to the first arrangement, wherein
the nucleic acid amplification reaction mixture contains a probe, and the probe contains an artificial nucleic acid.

2. The nucleic acid amplification method according to claim 1, wherein the probe has a larger binding force to a template nucleic acid than a probe containing only a natural nucleic acid.

3. The nucleic acid amplification method according to claim 1 or 2, wherein the artificial nucleic acid is LNA, 3'-amino-2',4'-BNA, 2',4'-BNA^{COC}, 2',4'-BNA^{NC}, PNA, GNA, or TNA.

4. The nucleic acid amplification method according to claim 3, wherein the artificial nucleic acid is LNA.

5. The nucleic acid amplification method according to any one of claims 1 to 4, wherein the probe is a labeled probe.

6. The nucleic acid amplification method according to claim 5, wherein the labeled probe is an RI-labeled probe, a fluorescence-labeled probe, or an enzyme-labeled probe.

7. The nucleic acid amplification method according to any one of claims 1 to 6, wherein the liquid has a specific gravity smaller than that of the nucleic acid amplification reaction mixture.

8. The nucleic acid amplification method according to any one of claims 1 to 6, wherein the liquid has a specific gravity larger than that of the nucleic acid amplification reaction mixture.

9. A nucleic acid amplification reaction apparatus, comprising:
a fitting section capable of fitting a nucleic acid amplification reaction vessel filled with a nucleic acid amplification reaction mixture and a liquid which has a specific gravity different from that of the nucleic acid amplification reaction mixture and is immiscible with the nucleic acid amplification reaction mixture;
a first heating section which heats a first region of the nucleic acid amplification reaction vessel to a first temperature;
a second heating section which heats a second region of the nucleic acid amplification reaction vessel to a second temperature; and
a driving mechanism which switches over between a first arrangement in which the first region is located lower than the second region in the direction of the gravitational force and a second arrangement in which the second region is located lower than the first region in the direction of the gravitational force, wherein
the nucleic acid amplification reaction mixture contains a probe, and the probe contains an artificial nucleic acid.

10. The nucleic acid amplification reaction apparatus according to claim 9, wherein the liquid has a specific gravity smaller than that of the nucleic acid amplification reaction mixture.

11. The nucleic acid amplification reaction apparatus according to claim 9, wherein the liquid has a specific gravity larger than that of the nucleic acid amplification reaction mixture.


SEQUENCE LISTING

<110> Seiko Epson Corporation

<120> Method for amplifying nucleic acid

<130> J018336601

<160> 4

<170> PatentIn version 3.5

<210> 1
<211> 22
<212> DNA
<213> Artificial Sequence

<220>
<223> PCR forward primer

<400> 1
atccaggtac gggtgaagac ac                                                22

<210> 2
<211> 23
<212> DNA
<213> Artificial Sequence

<220>
<223> PCR reverse primer

<400> 2
cgcatcaaca agtcctagcg aac                                               23

<210> 3

<211> 15

<212> DNA

<213> Artificial Sequence

<220>

<223> PCR forward primer

<400> 3

cgggacggaa agacc 15

<210> 4

<211> 15

<212> DNA

<213> Artificial Sequence

<220>

<223> PCR probe

<220>

<221> misc_feature

<222> 8

<223> 2',4'-BNA(LNA)-G(guanine)

<220>

<221> misc_feature

<222> 9

<223> 2',4'-BNA(LNA)-A(adenine)

<220>

<221> misc_feature

<222> 10

<223> 2',4'-BNA(LNA)-A(adenine)

<400> 4

cgggacgnnn agacc                                                    15

SEQUENCE LISTING

<110> Seiko Epson Corporation

<120> Method for amplifying nucleic acid

<130> J018336601

<160> 4

<170> PatentIn version 3.5

<210> 1
<211> 22
<212> DNA
<213> Artificial Sequence

<220>
<223> PCR forward primer

<400> 1
atccaggtac gggtgaagac ac                                                22

<210> 2
<211> 23
<212> DNA
<213> Artificial Sequence

<220>
<223> PCR reverse primer

<400> 2
cgcatcaaca agtcctagcg aac                                               23

<210> 3
<211> 15
<212> DNA
<213> Artificial Sequence

<220>
<223> PCR forward primer

<400> 3
cgggacggaa agacc                                                        15

<210> 4
<211> 15
<212> DNA
<213> Artificial Sequence

<220>
<223> PCR probe

<220>
<221> misc_feature
<222> 8
<223> 2',4'-BNA(LNA)-G(guanine)

<220>

<221> misc_feature
<222> 9
<223> 2',4'-BNA(LNA)-A(adenine)

<220>
<221> misc_feature
<222> 10
<223> 2',4'-BNA(LNA)-A(adenine)

<400> 4
cgggacgnnn agacc                                                        15